# EUROPEAN PATENT APPLICATION

(11) **EP 4 129 368 A1**
(43) Date of publication of application: **08.02.2023**
(21) Application number: 21189836.6
(22) Date of filing: 05.08.2021
(51) Int. Cl.: A61M 5/32, A61M 25/00

(54) **SAFETY DEVICE FOR A NEEDLE AND SAFETY NEEDLE SYSTEM COMPRISING A SAFETY DEVICE**

(71) Applicant: Aesculap AG, 78532 Tuttlingen (DE)
(72) Inventor: Koller, Tobias, 78532 Tuttlingen (DE)
(74) Representative: Patentanwälte Ruff, Wilhelm, Beier, Dauster & Partner mbB

(57) **Abstract**

The invention relates to a safety device (1000) for a needle with a housing (200) having a channel (201) with a proximal opening (210) and a distal opening (212) for a stem of a needle (100) to pass therethrough, wherein the housing (200) is configured to slide over the stem of the needle (100) such that the needle (100) is moveable relative to the housing (200) between a first needle position, in which the needle tip (101) protrudes from the distal opening of the housing (200) and a second needle position, in which the needle tip (101) is situated inside the channel (201), and with a closing device which is configured to close the distal opening of the housing (200) when the needle tip (101) is in the second needle position, wherein the closing device comprises a protection flap (8), wherein the protection flap (8) closes the distal opening when protection flap (8) is in a first protection flap position, wherein the closing device comprises a securing element, wherein the securing element is configured to block a movement or deformation of the protection flap (8) out of the first protection flap position towards the inside of the channel (201). The invention further relates to safety needle system comprising a needle (100) and a safety device /1000), and to a method of manufacturing a safety device (1000),

## Description

### TECHNICAL FIELD AND PRIOR ART

The invention relates to a safety device for a needle and to safety needle system comprising a needle and a safety device, and to a method of manufacturing a safety device.

Needles are used in medicine for an injection of fluids into a patient's body and/or for a withdrawal of fluids from the patient's body as well as for sewing muscle, fascia, and other materials. Needles have a sharp needle tip and represent a considerable risk of injuries for healthcare medical staff, especially after use when they are blood contaminated.

US2013/0172825 A1 shows safety needle system comprising a needle that is provided with a safety device. The needle has a distal end, a proximal end, and a stem extending therebetween, as well as a needle tip at the distal end thereof. The safety device is configured for sliding over the stem of the needle between a first position in a proximal area of the stem and a second position at the distal end of the stem. The safety device has a channel with a proximal opening and a distal opening for the needle stem to pass therethrough, wherein the needle tip is inside the channel when the safety device is in the second position. The safety device includes a closing device that closes the distal opening of the recess when the needle tip is inside the channel.

### SUMMARY OF THE INVENTION

It is the object of the invention to provide a safety device for a needle and a safety needle system comprising such a safety device, which is cost-efficient in production and allows a secure protection against injuries on a needle tip. It is a further object of the invention to provide a method for manufacturing a safety device.

This object is solved by the safety device, the safety needle system, and the method for manufacturing a safety device with the features of claims 1, 9, and 11. Preferred embodiments are defined in the dependent claims.

According to a first aspect, a safety device for protecting against injuries on a needle tip is provided, the safety device comprising a housing and a closing device, the housing having channel with a proximal opening and a distal opening for a stem of a needle to pass therethrough, wherein the housing is configured to slide over the stem of the needle such that the needle is moveable relative to the housing between a first needle position, in which the needle tip protrudes from the distal opening of the housing, and a second needle position, in which the needle tip is situated inside the channel, wherein the closing device is configured to close the distal opening of the housing when the needle tip is in the second needle position, wherein the closing device comprises a protection flap, wherein the protection flap closes the distal opening when protection flap is in a first protection flap position, wherein the closing device further comprises a securing element, which securing element is configured to block a movement or deformation of the protection flap out of the first protection flap position towards the inside of the channel.

Throughout this description and the claims, the indefinite article "a" or "an" means "one or more". Reference to "a first element" does not mandate presence of "a second element". Further, the expressions "first" and "second" are only used to distinguish one element from another element and not to indicate any order of the elements.

In the first needle position, the needle tip protrudes from the safety device and the needle is useable for example by sticking the needle into a patient's body. When removing the needle, the safety device is moved relative to the needle towards the needle tip. When the needle reaches the second needle position, the protection flap is moved into the first protection flap position and closes the distal opening of the housing. The housing is thus closed and any injury of the healthcare staff with the tip of the contaminated needle is excluded.

In accordance with the invention, a securing element is provided, wherein the securing element prevents an unintentional movement or deformation of the protection flap to an inside of the channel, which movement could result in an exposure of the needle tip.

In an embodiment, the protection flap is elastically bendable and forced by its restoration forces into or towards the first protection flap position, wherein when the protection flap is not blocked by the securing element, the protection flap is elastically bendable against its restoration force into the second protection flap position, in which second protection flap position the protection flap protrudes into the channel. In other words, the restoration forces act on the protection flap for holding the protection flap in the first protection flap position, in which the protection flap closes the distal opening of the channel. In one embodiment, when in the first needle position the needle loads the protection flap for holding the protection flap in the second protection flap position against the restoration forces of the protection flap. When moving the housing relative to the needle, the protection flap is released and moved by its restoration forces into or towards the first protection flap position. In one embodiment, when unloaded the protection flap assumes the first protection flap position. In other words, the first protection flap position in embodiments of the protection flap is its neutral position.

In one embodiment, the safety element is a safety latch provided at a sidewall of the channel, which for example is tapered to allow a unidirectional movement of the protection flap from a second protection flap position inside the channel into the first protection flap position and blocks a movement in the opposite direction.

In another embodiment, the securing element is a bendable and/or swivellable securing flap having a free end that projects into a movement path of the protection flap between the first protection flap position and the second protection flap position, wherein the protection flap and the securing flap are configured to interact for allowing a unidirectional movement of the protection flap from the second protection flap position towards the first protection flap position. The swivellable or bendable securing flap allows for a unidirectional movement using only the restoration force of the protection flap, and a blocking of the movement in the opposite direction.

In one embodiment, the securing flap is elastically bendable, wherein the securing flap in a steady position is arranged in a first securing flap position, in which the securing flap blocks the movement of the protection flap out of the first protection flap position towards the second protection flap position, and wherein the securing flap is elastically bendable against its restoration force into a second securing flap position allowing the movement of the protection flap from the second protection flap position towards the first protection flap position.

In one embodiment, the securing flap extends into the movement path of the protection flap between the first protection flap position and the second protection flap position, wherein when moving the protection flap from the second protection flap position towards the first protection flap position, the protection flap acts on the securing flap and forces the securing flap to bend against its restoration force into the second securing flap position. The protection flap and the securing flap are formed and arranged so that a free end of the protection flap acts on the securing flap when the protection flap moves towards the first protection flap position due to its restoration forces. Once the free end of the protection flap is moved beyond the free end of the securing flap, the protection flap no longer loads the securing flap, and the securing flap returns into the first securing flap position.

In one embodiment, a projection is provided at a sidewall of the housing opposite to the securing flap, which is configured for forcing the securing flap into the first securing flap position during assembly of the housing and the closing device, wherein in particular the securing flap is provided with a bevel flap, which bevel flap is configured to ensure that the securing flap slides over the projection during assembly. In an embodiment, the projection is a slope projection, especially if there is no bevel flap provided to the securing flap. A slope on the slope projection facilitates sliding of the bevel flap or the respective edge of the securing flap over the projection during introduction of the safety device into the housing.

In the first securing flap position, into which the slope projection forces the securing flap, the securing flap projects from the sidewall with an acute angle. Hence, the securing flap has a low bending stiffness against forces acting on the securing flap for forcing the securing flap towards the sidewall, and a high bending stiffness against forces acting in an axial direction of the channel.

Prior to the assembly of the closing device and the housing, the securing flap is not moved into the first securing flap position and is arranged in a position, in which it does not hinder the movement of protection flap. This allows introducing a needle and/or a hollow cylinder used for a subsequent introducing of the needle.

In one embodiment, an abutment portion is provided at the distal opening, wherein the protection flap in the first protection flap position abuts against the abutment portion. The abutment portion securely blocks a movement of the protection flap beyond the distal opening. Hence, the protection flap in the first protection flap position is clamped between the abutment portion and the securing element, in particular the securing flap.

In one embodiment, the closing device is an integral structure formed from a sheet metal piece. This allows for a manufacturing with low manufacturing costs. In addition, such a structure can be mounted to a housing formed as a single piece plastic component to further reduce the manufacturing costs.

According to a second aspect, a safety needle system comprising a needle and a safety device with a housing and a closing device are provided. The needle is accommodated in the safety device, such that the needle is moveable relative to the housing of the safety device between a first needle position, in which the needle tip protrudes from the distal opening of the housing and a second needle position, in which the needle tip is situated inside the channel, wherein prior to a use of the safety needle system, the needle inserted in the channel extends between the proximal end and the distal end of the channel and forces the protection flap out of the first protection flap position. A length of a needle part remaining inside the channel in the second needle position and the protection flap of the closing device are adapted to each other so that when moving the needle relative to the housing into the second needle position, the protection flap is released and moves into the first protection flap position.

The needle in embodiments of the invention is deformed for providing a stop against withdrawal of the needle from the safety device. In particular, in one embodiment, the needle is pressed in two opposing radial directions, causing a radial expansion in directions orthogonal thereto.

According to a third aspect, a method for producing a safety device is provided, wherein the closing device is formed by bending a sheet metal piece at three bending lines into a U-shaped form having a central portion, a first sidewall and a second sidewall extending orthogonal from the central portion, and a first base section extending parallel to the central portion at a distal end of the first sidewall, wherein prior to bending, a protection flap cut-out is cut out of the sheet metal piece at the first sidewall, which delimits a protection flap that after the bending extends in the extension of the first base section. In embodiments, the protection flap cut-out is U-shaped. The protection flap in embodiments is designed to have a length that is the same or slightly shorter than a length of the central portion so that the protection flap after bending closes an opening of the U-shaped form opposite to the central portion.

In addition, in an embodiment of the invention the closing device comprises a securing flap, wherein a securing flap cut-out is cut out of the sheet metal piece at the second sidewall, which delimits the securing flap having an axis at the bending line between the central portion and the second sidewall. The securing flap in embodiments is designed to extend over the entire length of the second sidewall. Prior to inserting the sheet metal piece into the housing, the securing flap is arranged flush with the second sidewall and does not hinder a mounting of a needle into the safety device. The needle forces the protection flap into the second protection flap position away. Upon inserting the closing device into the housing, the securing flap is forced into the first securing flap position by the projection provided at a sidewall of the housing opposite to the securing flap.

In an embodiment, the securing flap is provided with a bevel flap cut-out for forming a bevel flap having a free end that points towards the axis of the securing flap. When manufacturing the safety device, the bevel flap is non-elastically bent towards an inside of the U-shaped form, so that the free end points towards the opposite first side wall. As described above, the securing flap interacts with a projection, in particular a slope projection, provided at a sidewall of the housing opposite to the securing flap, which projection forces the securing flap into the first securing flap position upon assembly. The bevel flap functions as run-up bevel that ensures that the securing flap slides over the projection.

In an embodiment, the sheet metal piece is bent at four bending lines, wherein a second base section is provided that extends parallel to the central portion at a distal end of the second sidewall, wherein an abutment portion cut-out is cut out of the sheet metal piece at the second sidewall, which delimits an abutment portion, which after bending extends in the extension of the second base section. In such embodiment, the second base section is provided prior to the provision of the first base section.

The cut-outs in embodiments are formed by laser cutting. The cut-outs are formed prior to bending allowing for a cost-efficient manufacturing method of the closing device, which after bending at the four bending lines is inserted in the housing.

### BRIEF DESCRIPTION OF THE DRAWINGS

In the following, embodiments of the invention will be described in detail with reference to the drawings. Throughout the drawings, the same elements will be denoted by the same reference numerals.
- Fig. 1:: shows a safety system comprising a safety device and a needle in a cross-section;
- Fig. 2:: shows a housing of the safety device of Fig. 1 in a cross-section;
- Fig. 3:: shows the needle and the closing device of the safety device of Fig. 1 in a cross-section, wherein the needle is in a first needle position;
- Fig. 3A:: shows a detail of the needle in a cross-section perpendicular to Fig. 3;
- Fig. 4:: shows a sheet metal piece for forming a closing device of the safety device of Fig. 1 prior to bending;
- Fig. 5:: shows manufacturing steps for forming the closing device of Fig. 4 for the sheet metal piece shown in Fig. 4;
- Fig. 6:: shows a detail of the closing device and the housing during assembly;
- Fig. 7:: shows an introduction of a hollow cylinder into the closing device during assembly of the safety system;
- Fig. 8:: shows the safety device with a hollow needle introduced therein;
- Fig. 9:: shows the needle and the closing device of the safety device of Fig. 1 in a cross-section, shortly after moving the needle into a second needle position;
- Fig. 9A:: shows a detail of the needle in a cross-section perpendicular to Fig. 9;
- Fig. 10:: shows the needle and the closing device of the safety device similar to Fig. 9 during a movement of a protection flap of the closing device into a first protection flap position; and
- Fig. 11:: shows the needle and the closing device of the safety device similar to Fig. 10 after a movement of the protection flap into the first protection flap position, wherein the protection flap is secured by a securing flap.

### DETAILED DESCRIPTION OF EMBODIMENTS OF THE INVENTION

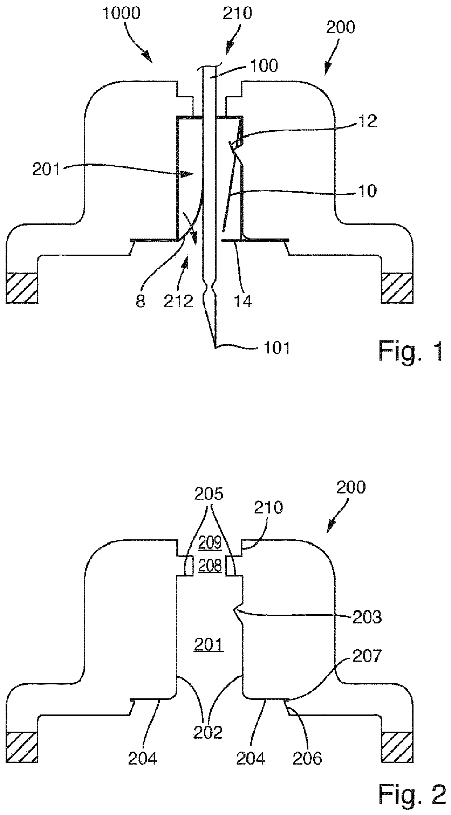

Fig. 1 shows a safety system 1000 comprising a needle 100 for insertion into a patient's body and a safety device. The safety device is configured for protecting against injuries on a tip 101 of a needle 100 during or after the needle 100 is pulled out of a patient's body. It comprises a housing 200 and a closing device, which is accommodated in the housing 200.

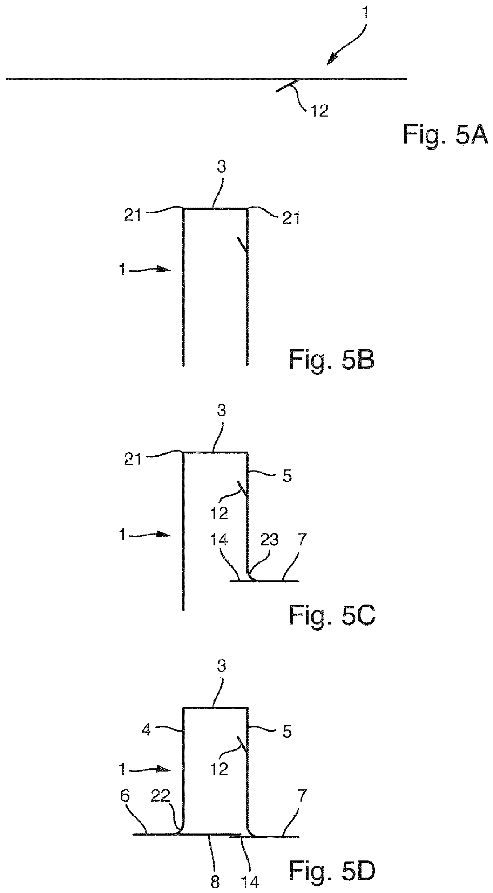

The housing 200 has channel 201 with a proximal opening 210 and a distal opening 212 for a stem of the needle 100 to pass therethrough. The needle 100 is moveable relative to the housing 200 between a first needle position shown in Fig. 1, in which the needle tip 101 protrudes from the distal opening 212, and a second needle position (see Fig. 5), in which the needle tip 101 is situated inside the housing 200.

The closing device is accommodated in the channel 201. It comprises an elastically bendable protection flap 8, wherein in an unloaded or neutral position, referred to as first protection flap position, the protection flap 8 closes the distal opening 212. The protection flap 8 is bent against its restoration force into a second protection flap position shown in Fig. 1, wherein the needle 100 holds the protection flap in the second protection flap position. When the needle is withdrawn, the restoration force causes a movement of the protection flap 8 back into the first protection flap position as indicated by an arrow in Fig. 1.

The closing device shown in Fig. 1 further comprises a securing flap 10 and an abutment portion 14, wherein as will be explained in more detail below, after a movement of the needle 100 into the second needle position, the protection flap 8 can be clamped between the securing flap 10 and the abutment portion 14 such that a movement of the protection flap 8 into the channel 201 or to an outside away from the channel 201 is blocked.

The closing device further comprises a bevel flap 12, the function of which will be explained in more detail below.

Fig. 2 shows the housing 200 of the safety device in isolation. The housing 200 is in one embodiment made of plastic. The housing 200 has a channel 201, which is connected with a channel 208 provided in a top wall 205. At the top wall, in the embodiment shown, a receiving section 209 is provided.

The channel 201 has left and right sidewalls 202. In one of the sidewalls 202, a slope projection 203 is formed. The channel 201 extends from the top wall 205 to a base wall 204.

On both sides of the channel 201, sloped walls 206 protrude from the base wall 204, wherein undercuts 207 are formed at transition regions between the base wall 204 and the sloped walls 206.

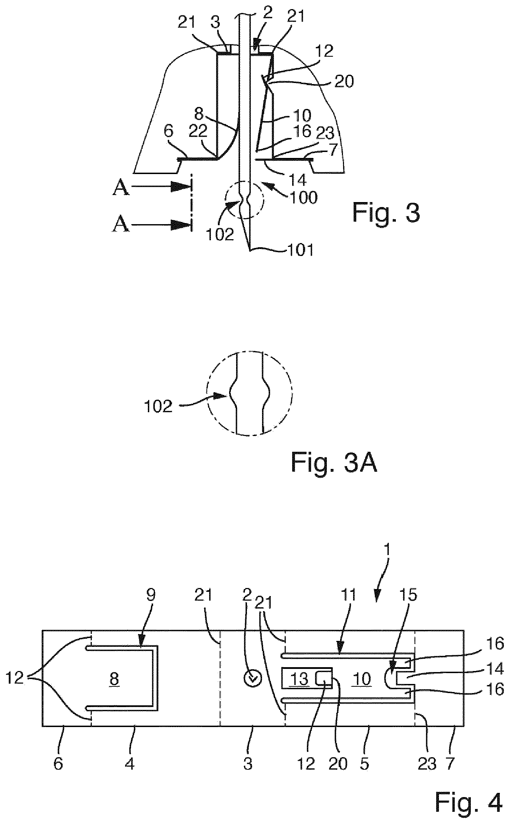

The closing device to be inserted into the housing 200 is described in more detail with reference to Figs. 3 to 5.

In the embodiment shown, the closing device is a sheet metal piece 1 formed by laser cutting and bending.

Fig. 3 shows the sheet metal piece 1 together with a needle 100, wherein the needle is in the first needle position.

For avoiding that the needle 100 is pulled out of the safety device, a stop portion 102 is formed on the needle 100 above the needle tip 101.

In the embodiment shown, the stop portion 102 of the needle 100 is formed by pressing the needle 100 in a radial direction from two opposing sides. Thereby, the diameter of the needle 100 is reduced in the pressing direction and, in turn, as shown in Fig. 3A is increased in a direction of the cross section of needle 100 which is orthogonal to the pressing direction, wherein a diameter of the needle through hole 2 is smaller than the increased diameter of the needle 100. The increased diameter thus forms a stop portion 102, which avoids that the needle 100 is pulled out of the safety device through the needle through hole 2 of the sheet metal piece 1.

Fig. 4 shows the sheet metal piece prior to bending. The sheet metal piece 1 is non-elastically bent about two bending lines 21 for forming a central portion 3 and two parallel sidewalls 4, 5 extending orthogonal to the central portion 3. At the distal ends of the sidewalls 4, 5 a first base portion 6 and a second base portion 7 are provided, which are non-elastically bent with respect to the sidewalls 4,5 about bending lines 22, 23 to extend in parallel to the central portion 3.

A needle through hole 2 is provided in the central portion 3.

As shown in Fig. 4, by laser cutting a protection flap cut-out 9, a securing flap cut-out 11, a bevel flap cut-out 13, and an abutment portion cut-out 15 for the protection flap 8, the securing flap 10, the bevel flap 12, and the abutment portion 14, respectively are provided. The protection flap cut-out 9 is shaped for creating the protection flap 8 having an axis that coincides with the bending line 22, a free end facing towards the central portion 3, and a length slightly shorter than a length of the central portion. The securing flap cut-out is shaped for creating a flap having an axis that coincides with the bending line 21 between the central portion 3 and the second sidewall 5. The securing flap 10 is formed to extend over the full length of the sidewall 5. The bevel flap 12 is provided on the securing flap 10, wherein a free end of the bevel flap 12 points towards the bending line 21 between the central portion 3 and the second sidewall 5. The abutment portion cut-out 15 is shaped for creating the abutment portion 14 in the extension of the second base portion 7.

After cutting, the sheet metal piece 1 with the cut-outs 9, 11, 13, 15 is bent along the bending lines 21, 22, 23 for creating the three-dimensional shape of the sheet metal piece 1 functioning as the closing device.

A bending process is described in more detail with reference to Fig. 5.

In a first step shown in Fig. 5(A). the bevel flap 12 is bent along a bevel flap bending line 20 so that the distal end the bevel flap 12 of the final product protrudes towards an inside of the sheet metal piece 1 facing towards the central portion 3.

In a second step shown in Fig 5(B), the sheet metal piece 1 is non-elastically bent along the central portion bending lines 21 to create the first sidewall 4 and the second sidewall 5 on both sides of the central portion 3 to protrude orthogonal to the central portion.

In a third step shown in Fig. 5(C), the second sidewall 5 is non-elastically bent along the second base portion bending line 23 to create the second base portion 7. Thereby, as the abutment portion 14 is an extension of the second base portion 7, the abutment portion 14 is swiveled towards the first sidewall 4 and projects to the inside of the safety sheet 1.

In a fourth and last bending step shown in Fig. 5(D), the first sidewall 4 is bent along the first base portion bending lines 22 to create the first base portion 6. Thereby, as the protection flap 8 is an extension of the first base portion 6, the protection flap 8 projects towards the second sidewall 5 and the lower surface of the distal end of the protection flap 8 comes into abutment with the upper side of the abutment portion 14.

The bent safety sheet 1 is mounted to the housing 200 shown in Fig. 2.

When the bent sheet metal piece 1 is mounted to the housing 200, the central portion 3 of the safety sheet 1 is inserted into the channel 201 and the sheet metal piece 1 is pushed from below into the channel 201 of the housing 200 until the central portion 3 of the sheet metal piece 1 abuts the top wall 205 and the first and second base portion 6, 7 of the sheet metal piece 1 abut against a base wall 204.

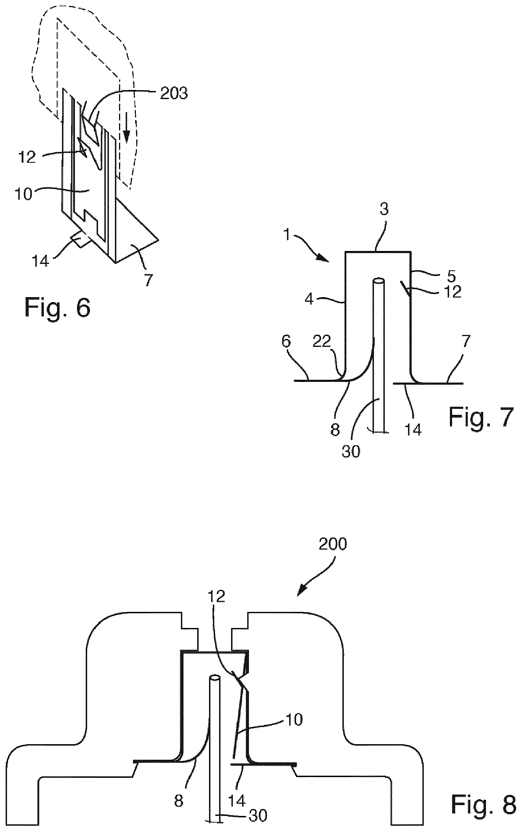

As schematically shown in Fig. 6. during the insertion of the sheet metal piece 1 into channel 201 of housing 200, the securing flap 10 with the bevel flap 12 comes into abutment against the slope projection 203. The bevel flap 12 facing the slope projection 203 slides over the slope projection 203. Then, when the bevel flap 12 slides over the slope projection 203, the securing flap 10 is bent about the central portion bending 21 of the second sidewall 5 towards the central axis of channel 201.

At the end of the introduction of the sheet metal piece 1 into the channel 201 of the housing 200, the side edges of the first base portion 6 and the second base portion 7 slide along the sloped walls 206 of the housing 200. Thereby, the sidewalls 4, 5 may also elastically swivel into channel 201. At the end of the sloped walls 206, the undercuts 27 are formed into which the side edges of the first base portion 6 and second base portion 7 enter, respectively, when the first base portion 6 and the second base portion 7 cone into abutment against the base wall 204. Thereby, the sheet metal piece 1 is secured within the housing 200 and the positions of the sidewalls 4, 5 are restored.

Alternatively to the sloped walls 206 and the undercuts 207, the sheet metal piece 1 in one embodiment is secured in the housing e.g. by deforming parts of the housing thermally or plastically so that the sheet metal piece 1 is fixed to the housing 201 by form-fit.

For introducing the needle 100 into the housing 200, the protection flap 8 of sheet metal piece 1 is elastically bent towards the sidewall 4 from below. As in the embodiment shown, as a bending is blocked by the securing flap 10 after an insertion of the sheet metal piece 1 into the housing 200, an insertion of the needle 100 has to be done before mounting the sheet metal piece 1 into the channel 201 of the housing 200.

As shown in Fig. 7 and 8, in one embodiment, prior to mounting the sheet metal piece 1 into the housing 200, a hollow mounting cylinder 30 is used for bending or pushing the protection flap 8 toward the second protection flap position so that the needle 100 can be introduced from above into a central lumen of this mounting cylinder. After the hollow mounting cylinder 30 is introduced into the bent sheet metal piece 1, the sheet metal piece 1 together with the hollow mounting cylinder 30 is mounted to the housing 200 as shown in Fig. 8, and the needle 100 is introduced. After introduction of the needle 100 into the housing 200, the mounting cylinder can be removed and the protection flap 8 bears against an outer wall of the needle 100. Thereby, the needle 100 blocks a movement of the protection flap 8 back into the first protection flap position.

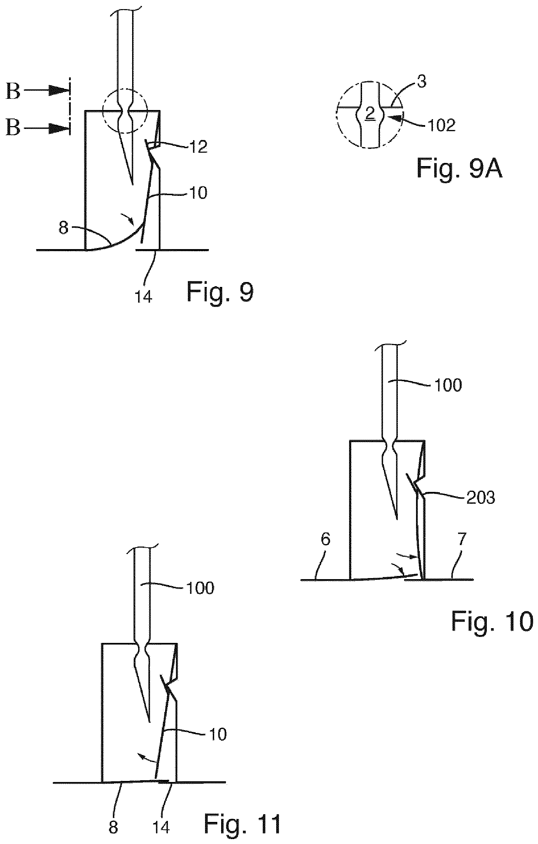

The working principle of the safety needle is described in more detail with reference to Figs. 9 to 11.

For pulling the needle 100 out of the patient's body, the housing 200 is held in position on the patient's skin. Then the needle 100 is pulled relative to the housing 200 out of the patient's body.

As shown in Fig. 9A, a movement of the needle 100 relative to the housing is restricted by the stop 102 formed on the needle 100.

Upon a movement of the needle 100 relative to the housing 200, the needle tip 101 passes a free end of the protection flap 8 and releases the protection flap 8.

After its release, the protection flap 8 is moved due to its restoration forces into the first protection flap position, that is in the embodiment shown, it is swiveled towards a horizontal position in the drawing plane, i.e., a position at least essentially in parallel to the patient's skin. Upon the movement, a free end of the protection flap 8 abuts against the securing flap 10. A restoration force of the securing flap 10 forces the securing flap 10 into the first securing flap position shown in Fig. 9, in which its free end is distant from a sidewall 5 and protrudes into the movement path of the protection flap 8. A restoration force of the protection flap 8 is larger than a restoration force of the securing flap 10. Therefore, when the free end of the protection flap 8 contacts an inner surface of securing flap 10, the securing flap 10 is urged elastically towards the sidewall 5 of the safety sheet 1. With the movement of the securing flap 10 to the sidewall 5, the free end of protection flap 8 slides along the inner surface of securing flap 10 towards the first protection flap position.

Lengths and swivel axes of the protection flap 8 and the securing flap 10 are configured such that shortly before reaching the first protection flap position, the free end of the protection flap 8 passes the free end of the securing flap 10. At this instant, as show in Figs. 10 and 11, the securing flap 10 is no longer urged by the protection flap 8 and returns into its original position, i.e., the first securing flap position shown in Figs. 1, 3, and 6.

The above described motions of the protection flap 8 and the securing flap 10 give a clear acoustic and haptic feedback to the practitioner that the safety device is active and no injury on the needle tip is possible, even before the safety needle is removed from the patient's skin. This provides a big benefit to the practitioner in comparison to other safety devices for needles, where activation of the safety device is mostly noticeable merely visually after removing the safety needle from the patient's skin.

In the embodiment shown, the safety device further comprises the abutment portion 14, wherein when the protection flap 8 is in the first protection flap position as shown in Fig. 11, the protection flap 10 abuts against the abutment portion 14.

Hence, when the securing flap 10 is in the first securing flap position and the protection flap 8 is in the first protection flap position as shown in Fig. 11, the protection flap 8 may neither be pushed into the channel 201 nor to the outside to protrude from the channel 201 but is fixedly held in the first protection flap position between the securing flap 10 and the abutment portion14.

In an embodiment not shown, additional undercuts are provided at the top wall 205 of channel 201 in a direction transversal to the longitudinal direction of the safety sheet 1. The undercuts allow to fix the safety sheet 1 more firmly within the channel 201 of the housing 200 when the needle 100 pushes the securing flap 8 downwards in order to avoid that the central portion 3 of the safety sheet 1 is separated from the top wall 205 of the channel 201.

In alternative or in addition, in another embodiment not shown, a valve or a septum are placed into the receiving section 209 of the housing. This valve or septum avoids that blood which attaches to the outer surface of the needle, is exposed to an area where a practitioner may come into contact with it. In one embodiment, in order to further secure the valve or septum within the receiving section 209, the sidewalls 210 of the receiving section 209 are inclined so that the receiving section has the shape of a truncated cone with the smaller diameter on the outer side of the receiving section. In another embodiment, a step is provided in the sidewall for securing the valve or septum.

In the embodiments shown, the housing is a one-piece design, in which the safety sheet 1 is held within the housing 200 using the undercuts 207. In an alternative embodiment, a housing having a two-piece similar to US 2013/0172825 A1 is provided, in which the safety sheet 1 is clamped.

## Claims

1. Safety device for protecting against injuries on a needle tip,
with a housing (200) having a channel (201) with a proximal opening (210) and a distal opening (212) for a stem of a needle (100) to pass therethrough, wherein the housing (200) is configured to slide over the stem of the needle (100) such that the needle (100) is moveable relative to the housing (200) between a first needle position, in which the needle tip (101) protrudes from the distal opening of the housing (200) and a second needle position, in which the needle tip (101) is situated inside the channel (201), and
with a closing device which is configured to close the distal opening of the housing (200) when the needle tip (101) is in the second needle position, wherein the closing device comprises a protection flap (8), wherein the protection flap (8) closes the distal opening when protection flap (8) is in a first protection flap position,
**characterized in that** the closing device comprises a securing element, wherein the securing element is configured to block a movement or deformation of the protection flap (8) out of the first protection flap position towards the inside of the channel (201).

2. The safety device according to claim 1, **characterized in that** the protection flap (8) is elastically bendable and forced by its restoration forces into or towards the first protection flap position, wherein when the protection flap (8) is not blocked by the securing element (10), the protection flap (8) is elastically bendable against its restoration force into a second protection flap position, in which second protection flap position the protection flap (8) protrudes into the channel (201).

3. The safety device according to claims 1 or 2 **characterized in that** securing element is a bendable and/or swivellable securing flap (10) having a free end that projects into a movement path of the protection flap (8) between the first protection flap position and the second protection flap position, wherein the protection flap (8) and the securing flap (10) are configured to interact for allowing a unidirectional movement of the protection flap (8) from the second protection flap position towards the first protection flap position.

4. The safety device according to claim 3, **characterized in that** the securing flap (10) is elastically bendable, wherein the securing flap (10) in a steady position is arranged in a first securing flap position, in which the securing flap (10) blocks the movement of the protection flap (8) out of the first protection flap position towards the second protection flap position, and wherein the securing flap (10) is elastically bendable against its restoration force into a second securing flap position allowing the movement of the protection flap (8) from the second protection flap position towards the first protection flap position.

5. The safety device according to claim 4, **characterized in that** the securing flap (10) extends into a movement path of the protection flap (8) between the first protection flap position and the second protection flap position, wherein when moving the protection flap towards the first protection flap position, the protection flap (8) acts on the securing flap (10) and forces the securing flap (10) to move against its restoration force into the second securing flap position.

6. The safety device according to claim 5, **characterized in that** a projection, in particular a slope projection (203), is provided at a sidewall (202) of the housing (200) opposite to the securing flap (10), which projection is configured for forcing the securing flap (10) into the first securing flap position during assembly of the housing (200) and the closing device, wherein in particular the securing flap (10) is provided with a bevel flap (12), which bevel flap (12) is configured to ensure that the securing flap (10) slides over the projection during assembly.

7. The safety device according to any one of claims 1 to 6, **characterized in that** an abutment portion (14) is provided at the distal opening, wherein the protection flap (8) in the first protection flap position abuts against the abutment portion (14).

8. The safety device according to any one of claims 1 to 7, **characterized in that** the closing device is an integral structure formed from a sheet metal piece (1).

9. A safety needle device comprising a needle (100) and a safety device according to any one of claims 1 to 8.

10. The safety needle device according to claim 9, **characterized in that** the needle is deformed for providing a stop against withdrawal of the needle from the safety device.

11. A method for producing a safety device according to any one of claims 1 to 8, wherein the closing device having the protection flap (8) is formed by bending a sheet metal piece (1) at three bending lines (21, 22, 23) into a U-shaped form having a central portion (2), a first sidewall (4) and a second sidewall (5) extending orthogonal from the central portion (2), and a first base section (6) extending parallel to the central portion (2) at a distal end of the first sidewall (4), wherein prior to bending, a protection flap cut-out (9) is cut out of the sheet metal piece (1) at the first sidewall (4), which delimits the protection flap (8), which after bending extends in the extension of the first base section (6).

12. The method according to claim 11, **characterized in that** a securing flap cut-out (1) is cut out of the sheet metal piece (1) at the second sidewall (5), which delimits a securing flap (10) having an axis at the bending line (21) between the central portion (2) and the second sidewall (5).

13. The method according to claim 12, **characterized in that** the securing flap (10) is provided with a bevel flap cut-out (13) for forming a bevel flap having a free end that points towards the axis of the securing flap (10).

14. The method according to claim 11, 12 or 13, **characterized in that** the sheet metal piece (1) is bent at four bending lines (21, 22, 23), wherein a second base section (7) extends parallel to the central portion (2) at a distal end of the second sidewall (5), wherein an abutment portion cut-out (15) is cut out of the sheet metal piece (1) at the second sidewall (5), which delimits an abutment portion (14), which after bending extends in the extension of the second base section (7).

15. The method according to any one of claims 11 to 14, **characterized in that** the cut-outs (9, 11, 13, 15) are formed by laser cutting.
